Europäisches Patentamt

European Patent Office

Office européen des brevets

(19)

(11) Publication number: **0 370 584**
**A1**

(12) # EUROPEAN PATENT APPLICATION

(21) Application number: **89202961.2**

(51) Int. Cl.5: **A61M 1/36**

(22) Date of filing: **22.11.89**

(30) Priority: **23.11.88 NL 8802888**

(43) Date of publication of application:
**30.05.90 Bulletin 90/22**

(84) Designated Contracting States:
**AT BE CH DE ES FR GB GR IT LI LU NL SE**

(71) Applicant: **AKZO N.V.**
**Velperweg 76**
**NL-6824 BM Arnhem(NL)**

(72) Inventor: **Van Driessche, Petrus Johannes**
**Dorothea Maria**
**Burggraaf 2**
**NL-4564 DN St. Jansteen(NL)**

(74) Representative: **Sieders, René et al**
**AKZO N.V. Patent Department (Dept. CO)**
**P.O. Box 9300**
**NL-6800 SB Arnhem(NL)**

(54) **Filter and process for preparing a leucocyte-poor platelet suspension.**

(57) Filter and process for preparing a leucocyte-poor thrombocyte suspension. Use is made of a non-woven filter displaying hydrophobic properties as characterised by a Critical Entry Surface Tension (CEST) of at most 60 dynes/cm, the average pore size of the filter being 2 to 50 $\mu$m as measured by the Coulter Porometer.

EP 0 370 584 A1

fig.2

## Filter and process for preparing a leucocyte-poor platelet suspension.

The invention relates to a filter with filter material composed of fibres for preparing a leucocyte-poor platelet suspension by filtration of a leucocytes- and platelets-containing starting suspension. The invention also comprises a process for preparing a leucocyte-poor platelet suspension making use of said filter.

For a number of reasons there is an increasing need in present-day medical practice for thrombocyte or blood platelet concentrates prepared from blood. Often patients are repeatedly given transfusions of platelet concentrates. This is demonstrated in particular by the articles "Use of Polyester Filters for the Preparation of Leucocyte-Poor Platelet Concentrates" by E.L. Snyder, L. De Palma, and P. Napychank (see Vox Sang. 54:21-23-1988) and "Prevention of HLA Alloimmunization as a Result of Platelet Transfusions" by M. van Marwijk Kooy, A. Brand, K. Sintnicolaas and H.C. van Prooyen (see Ned. Tijdschr. Geneesk. 1988; 132, no. 17, pp. 758-761). As is explained in said publications, a platelet concentrate suitable for medical application, which generally consists of a suspension of blood plasma with a high concentration of platelets, can be prepared from blood by removing therefrom a large number of the leucocytes or white blood cells. Leucocytes can be removed from the blood by centrifugation or filtration. Since use of the centrifugation method is attended with a considerable loss of platelets, preference is given to a filtration method.

Said article by M. van Marwijk Kooy et al. describes the filtration of platelet concentrates of a group of donors through a filter filled with cotton wool, in which process there was an average loss of platelets of 19% and a highly favourable removal of leucocytes. Also, the article by M. van Marwijk Kooy proposes making use of a filter of cellulose-acetate fibres, the platelets in the starting suspension having been treated with prostacycline. Favourable removal of leucocytes is obtained both by the use of the cotton wool filter and by that of the cellulose-acetate filter with the platelets having been treated with prostacycline prior to filtration, so that the absolute number of leucocytes in platelet concentrates of 6 random donors is nearly always lower than $1 \times 10^7$ leucocytes per concentrate. The drawback to both the cotton wool and the cellulose-acetate filter, however, is that a loss of platelets of 19% is still quite considerable.

The possibility of preparing a leucocyte-poor platelet concentrate by filtration also is described in said article by E.L. Snyder et al. Testing the polyester fibres-based filter marketed under the trade mark Sepacell R-500A® resulted in the removal of about 92% of the leucocytes and about 98% of the platelets from a stored concentrate, which is unacceptable. Testing the polyester fibres-based filter marketed under the trade mark Travenol 4C2423®, although resulting in a loss of less than 1% of the platelets, led to a removal of only 14% of the leucocytes, which is not acceptable either.

The invention has for its object to provide a filter and a process of the type mentioned in the opening paragraph for the preparation of a leucocyte-poor platelet concentrate, in which the drawbacks to the known filters and processes have been overcome.

The filter according to the invention is characterized in that the filter material consists of one or more layers of interlaced fibres, more particularly in the form of a non-woven web, and at least 50% of the fibres forming the filter material, at least at their outside surface, display hydrophobic properties as characterized by a CEST (Critical Entry Surface Tension) of at most 60 dynes/cm, and the average size of the pores in the filter is 2-50 µm, measured by the Coulter® Porometer in accordance with the method defined in the specification. Preferably, according to the invention virtually all of the fibres forming the filter material have hydrophobic properties at their outside surface.

The extent to which a material may be said to be hydrophobic, hereinafter to be called "hydrophobicity", can be measured as follows. At first liquids of varying surface tensions are prepared. In each test three drops of 40 µl each are applied to the material in question, and after three minutes it is visually established whether or not the drops have spread out over the material. The test is repeated with liquids of decreasing surface tension until the drops have just started to spread out. The surface tension of the test liquid at which the liquid just starts to spread out is taken as the so-called Critical Entry Surface Tension (CEST), "Entry" referring to the drops entering the pores of the materal. The higher the "hydrophobicity" of the material, the lower the CEST needed to wet it.

In a preferred embodiment of the filter according to the invention the fibres with hydrophobic surface properties consist of a hydrophobic material, such as polyolefins, preferably polypropylene. Alternatively, the fibres with hydrophobic surface properties in the filter according to the invention may be hydrophilic with only the outside surface portion having hydrophobic properties. Favourable results can be obtained when the filter according to the invention is characterized in that the average size P of the pores in the filter is 3 to 25 µm, preferably 5 to 15 µm, and the average diameter of the fibres is 0,1 to 25 μm, preferably 1 to 5 μm. The non-woven web is preferably of the so-called melt-blown type, in the production of which a large number of molten polymer filaments are extruded from one or more spinneret plates, drawn to very thin filaments at a high rate using a fluid, for instance air, and deposited on a moving carrier, the still plastic filaments sticking together at their cross-over points to form a non-woven web. An effective embodiment is characterized according to the invention in that the filter consists of a non-woven web, the filter according to the invention generally being composed of at least 10, more particularly 20-40 superimposed non-woven webs, each of a thickness of 0,1 to 20 mm, the total thickness of all the non-woven layers combined being 2 to 20 mm. A favourable embodiment of the filter according to the invention is characterized in that the bulk density of the total fibre mass of the filter is 0,02 to 0,8 g/cm³, preferably 0,1 to 0,6 g/cm³. Said bulk density is obtained by dividing the weight of the total mass of fibres serving as filter by the volume of the space containing said total mass of fibres. The outer circumference of the non-woven web layers forming the filter is with advantage such as will give a surface area for each of the superimposed non-woven layers of 5 to 80 cm².

The invention further relates to a filtering apparatus in which the filter, together with a storage reservoir for the starting suspension and a collecting reservoir for the leucocyte-poor platelet suspension, forms a closed system. The filter, the storage reservoir, and the collecting reservoir may be interconnected, for instance by sterile couplings.

The invention also comprises a process for preparing a leucocyte-poor platelet concentrate, making use of the described filter according to the invention.

It should be noted that European Patent Application 267 286 also describes a fibre filter for the preparation of a platelet concentrate from a suspension containing leucocytes and platelets. In this process use is made of fibres of polyethylene terephthalate (PETP) of a diameter of 1,8 m coated with a special surface layer. Of the 11 Examples provided in this patent application the most favourable result is attained using the procedure according to Example 10, in which 100% of the leucocytes were removed from the starting suspension and the platelet passage rate was 93,8%. According to the remaining Examples the percentages of removed leucocytes and passed platelets, respectively, were in the range of 86,1 to 98,6 and 76,4 to 92,3%, respectively.

It should also be added that European Patent Application 0 155 003 describes a fibre filter for a different purpose, viz. the preparation of an erythrocyte concentrate, in which process the highest possible proportion of leucocytes is to be removed from the starting suspension. Such a filter is composed of a prefilter and a main filter. The prefilter serves to remove the coarser particles contained in the starting supension, which are formed by clotting blood cells.

The main filter serves to remove the leucocytes.

According to the Examples the polymers used for the prefilter are polyester, cuprammonium, and polypropylene and for the main filter polyester, nylon 66, and polyacrylate.

A commercial embodiment of the filter according to this European Patent Application 0155003 is the filter referred to above as Sepacell R-500A®.

The invention will be further illustrated with reference to the schematic drawings.

Figs. 1 and 2 show in plan view and in cross-section, respectively, a filter according to the invention placed in a filter housing.

Fig. 3 shows a closed system in which the fibre filter is incorporated.

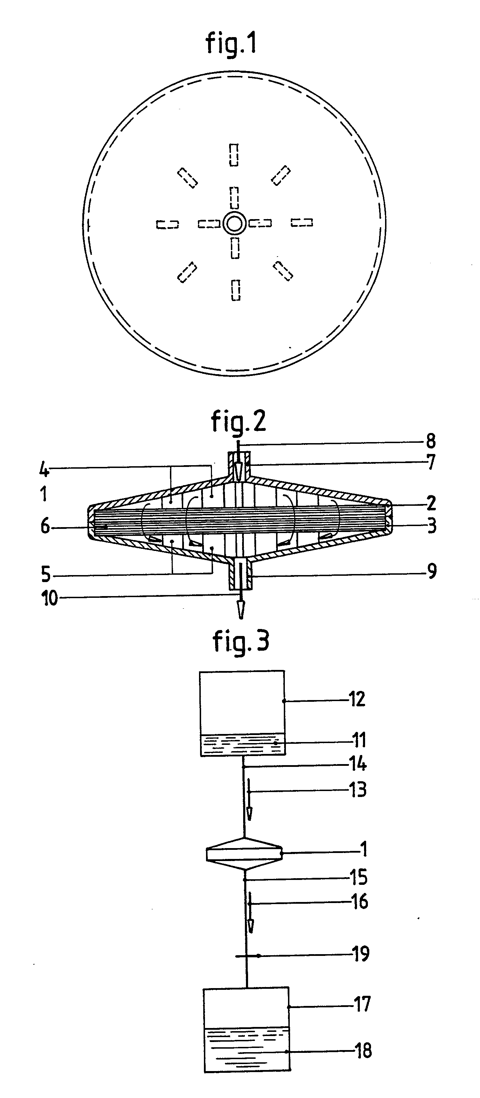

The filter generally indicated with the numeral 1 in Fig. 1 consists of a bipartite housing, of which the upper half 2 and the lower half 3 are fixed together. Both the upper half and the lower half of the housing are provided internally with a number of narrow clamping ribs 4 and 5, respectively, by which the stack of for instance 32 non-woven filter discs of polypropylene fibres indicated with 6 may be sufficiently compressed. The total thickness of the 32 filter discs may for instance be 6 mm at a diameter of, say, 70 mm. The upper half 2 of the housing is provided with an inlet port 7, through which the starting suspension may be introduced in the direction of the arrow 8. The lower half 3 of the filter housing is provided with an outlet port 9 through which the end product in the form of a platelet concentrate can be removed from the filter in the direction of the arrow 10.

Fig. 3 is a schematic illustration of a construction with a closed system according to the invention for the preparation of a platelet concentrate. The starting suspension 11, in the form of a blood plasma suspension containing a large amount of leucocytes and platelets is contained in the storage reservoir 12 in the form of a plastic bag. From the reservoir 12 the suspension flows by gravity along the direction of the arrow 13 through a plastic tube 14 to the filter 1. In the above-described manner the leucocytes are to a very large extent entrapped in the filter 1, and virtually all platelets are passed. The end product 18 is removed through the plastic tube 15 in the direction of the arrow 16 to its collecting reservoir 17, which is also formed by a plastic bag. The storage reservoir 12, the filter 1, and the collecting reservoir 17 may optionally be interconnected by sterile couplings.

After the full contents of the storage reservoir 12 have been filtered, the plastic tube 15 is sealed at 19, after which the tube 15 may be severed upstream of 19.

The average size P of the pores in a fibre layer is measured using the Coulter® Porometer manufactured by Coulter Electronics Ltd., Northwell Drive, Luton, Beds. LU 33 RH England. It is described in "Reference Manual for the Coulter Porometer (software level 1.3)," Issue B: November 1986, Part number 9903173. The average size P of the pores in the filter mentioned in the specification and the claims was measured in accordance with ASTM F316-80 (1980).

The measurement of the average diameter of the fibres is to be carried out in a statistically reliable manner, i.e., both as regards the number of fibres and their position in the filter.

In testing the filter according to the invention use was made of a web of polypropylene having an average pore size P = 12 $\mu$m, measured with the Coulter® Porometer. The measured CEST of the polypropylene used was 31 dynes/cm. The filter consisted of a 6 mm thick stack of 32 plies of the polypropylene web, with a diameter of 70 mm. The bulk density of the filter was 0,18 g/cm$^3$. The average diameter of the fibres determined by the EM method (Electron Microscope) was 2,4 $\mu$m.

So as to wet the hydrophobic material a physiological salt solution can be sucked or pressed through the filter by applying vacuum at its output or pressure at its input, respectively, or, alternatively, the filter can be pre-rinsed by a wetting solution such as, e.g., ethanol. In the latter case, the ethanol must be carefully rinsed out. Prior to the filtration the starting platelet concentrate contained 8,5 * 10$^5$ platelets per l and 2,6 * 10$^3$ leucocytes per $\mu$l. After the filtration the filtrate was found to contain 99,1% of the original number of platelets and only 0,3% of the original number of leucocytes.

In other words, use of the very simple filter according to the invention permits removing virtually all leucocytes from the suspension even without any special surface treatment of the fibres, the loss of platelets being less than 1%. Therefore, because of the very small loss of platelets the filter and the process according to the invention permit particularly efficient operation. Another especially important aspect of the filter and the process according to the invention consists in that the very strong reduction of the number of leucocytes in the leucocytes- and platelets-containing starting suspension to be processed results in an end product in the form of a virtually leucocyte-free platelet concentrate capable of meeting the very high standards set for medical transfusion practice.

Within the scope of the invention various modifications may be made. The fibres of which the filter consists may be continuous filaments or relatively short fibres. Also, instead of using a filter consisting of a number of axially stacked layers there may be employed a cylindrical filter through which the starting suspension is passed in radial direction. Such a cylindrical filter may for instance be obtained by making use of the process described in U.S. Patent Specification 4731 215.

As stated hereinbefore, the end product in the form of a platelet concentrate to be prepared using the filter and the process according to the invention should contain as few leucocytes as possible. Although actually no erythrocytes or red blood cells should be present in the end product to be prepared according to the invention, they are not detrimental to the envisaged applications, i.e., platelet transfusion. The filter according to the invention is intended for single use. The end product in the form of a platelet concentrate prepared according to the invention is usually administered to patients in transfusions in an amount which may vary case by case. As a rule, not more than 750 ml will be administered per transfusion. The minimum amount administered per transfusion will usually be 25 ml. In actual practice, however, the amount of platelet concentrate will generally be about 300-400 ml per transfusion.

As a rule, in the preparation according to the invention of the end product use is made of a starting platelet concentrate in the form of a suspension of blood plasma or a different liquid containing platelets, leucocytes, and erythrocytes. The hematocryt (a measure of the amount of red blood cells) of such a suspension usually will not be higher than 10%.

## Claims

1. A filter with filter material composed of fibres for preparing a leucocyte-poor platelet suspension by filtration of a leucocytes-and platelets-containing starting suspension, characterized in that the filter material consists of one or more layers of interlaced fibres, for instance in the form of a nonwoven web, and at least 50% of the fibres forming the filter material, at least at their outside surface, display hydrophobic properties as characterized by a CEST (Critical Entry Surface Tension) of at most 60 dynes/cm and the average size P of the pores in the filter is 2-50 $\mu$m, measured by the Coulter® Porometer in accordance with the method defined in the specification.

2. A filter according to claim 1, characterized in that virtually all of the fibres forming the filter material have hydrophobic properties at their outside surface.

3. A filter according to claim 1 or claim 2, characterized in that the fibres with hydrophobic surface properties wholly consist of a hydrophobic material, such as polyolefin.

4. A filter according to claim 3, characterized in that the hydrophobic fibres consist of polypropylene.

5. A filter according to claim 1, characterized in that the fibres with hydrophobic surface properties are of a hydrophilic material, with only the outside surface portion having hydrophobic properties.

6. A filter according to claim 1, characterized in that said average size P of the pores in the filter is 3 to 25 m, more particularly 5 to 15 m.

7. A filter according to claim 1, characterized in that the average diameter of the fibres is 0,1 to 25 m, more particularly 1 to 5 m.

8. A filter according to claim 1, characterized in that the filter consists of a non-woven web of the melt-blown type.

9. A filter according to claim 8, characterized in that the filter is composed of at least 10, more particularly 20-40 superimposed nonwoven webs, each of a thickness of 0,1 to 20 mm, and the total thickness of all the non-woven layers combined is 2 to 20 mm.

10. A filter according to claim 1, characterized in that the bulk density of the total fibre mass of the filter is 0,02 to 0,8 g/cm$^3$, preferably 0,1 to 0,6g/cm$^3$.

11. A filter according to claim 9, characterized in that the outer circumference of the non-woven layers forming the filter is such as will give a surface area for each of the non-woven layers of 5 to 80cm$^2$.

12. An apparatus equipped with a filter according to any one of the claims 1 through 11, characterized in that the filter, together with a storage reservoir for the starting suspension and a collecting reservoir for the leucocyte-poor platelet suspension, forms a closed system.

13. A process for preparing a leucocyte-poor, platelets-containing suspension by filtration of a leucocytes- and platelets-containing suspension making use of a filter composed of fibres, characterized in that use is made of a filter in which at least 50% of the fibres forming the filter material display hydrophobic properties at least at their outside surface, and the average size P of the pores in the filter is 2 to 50 $\mu$m, measured by the Coulter® Porometer in accordance with the method indicated in the specification the filter material used consisting of one or more layers of interlaced fibres, for instance in the form of a non-woven web.

14. A process according to claim 13, characterized in that use is made of a filter in which virtually all of the fibres forming the filter material display hydrophobic properties at their outside surface.

15. A process according to claim 13 or claim 14, characterized in that use is made of a filter in which the fibres with hydrophobic surface properties wholly consist of a hydrophobic material, such as polyolefin, more particularly polypropylene.

16. A process according to claim 13, characterized in that use is made of a filter in which the fibres with hydrophobic surface properties are hydrophilic, with only the outside surface portion having hydrophobic properties.

17. A process according to claim 13, characterized in that use is made of a filter in which the average size P of the pores is 3 to 25 m, more particularly 5 to 15 $\mu$m.

18. A process according to claim 13, characterized in that use is made of a filter in which the average diameter of the fibres is 0,1 to 25 $\mu$m, more particularly 1 to 5 $\mu$m.

19. A process according to claim 13, characterized in that use is made of a filter in the form of a non-woven web of the melt-blown type.

20. A process according to claim 19, characterized in that use is made of a filter composed of at least 10, more particularly 20-40 superimposed non-woven webs, each of a thickness of 0,1 to 20 mm.

21. A process according to claim 13, characterized in that use is made of a filter in which the bulk density of the total fibre mass is 0,02 to 0,8 g/cm$^3$, preferably 0,1 to 0,6 g/cm$^3$.

22. A process according to claim 20, characterized in that use is made of a filter in which the outer circumference of the non-woven layers forming the filter is such as will give a surface area for each of the non-woven layers of 5 to 80 cm$^2$ and the thickness of all the non-woven layers combined is 2 to 20 mm.

# fig.1

# fig.2

# fig.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | EP-A-0 155 003 (ASAHI MEDICAL CO.) * Pg. 5, 1. 6-20; pg. 6, 1. 11-20; pg. 7, 1. 2-17 * | 1,2,3,6 ,10,13, 14,15, 17,18, 21,22 | A 61 M    1/36 |
| A | FR-A-2 419 073 (ASAHI KASEI KOGYO K.K.) * Pg. 3, 1. 2-13; pg. 4, 1. 1-17; pg. 5, 1. 27-38 * | 1,7,10, 13,17, 18,21 | |
| A | US-A-4 157 965 (D.A. RAIBLE) * Col. 2, 1. 59-61 * | 1-4,9, 13-15, 20 | |

TECHNICAL FIELDS SEARCHED (Int. Cl.5)

A 61 M
B 01 D

The present search report has been drawn up for all claims

| Place of search | Date of completion of the search | Examiner |
|---|---|---|
| THE HAGUE | 15-12-1989 | KERRES P.M.G. |